# EUROPEAN PATENT SPECIFICATION

(11) **EP 0 964 913 B1**
(45) Date of publication and mention of the grant of the patent: **31.10.2001**
(21) Application number: 97915789.8
(22) Date of filing: 05.03.1997
(51) Int. Cl.: C12M 1/26, C12M 1/28, C12M 1/32, C12M 1/34, C12Q 1/04, C12Q 1/58

(54) **DEVICE FOR URINE TESTING**
VORRICHTUNG ZUM TESTEN VON URIN
DISPOSITIF D'ANALYSE D'URINE

(43) Date of publication of application: 22.12.1999
(73) Proprietor: Gesta Diagnostic AB, 101 24 Stockholm (SE)
(72) Inventor: Davarzan, Hamid, 175 34 Järfälla (SE)
(74) Representative: Wagner, Karl Heinz
(86) International application number: SE9700378
(87) International publication number: WO9842821

(56) References cited:
- DE-A- 2 711 201
- SE-C- 503 177
- US-A- 4 054 491

## Description

### Technical field of the innovation

The existing innovation bears upon a device for resistance tests of in e.g. urine occurring bacteria at which the device constitutes of that kind that consists of a closable cylindrical reservoir and one in this located test device prepared with a culture medium. The test device is situated in the front part of one in the reservoir axially shiftable piston, which makes it watertight against the inner wall of the reservoir, at which one of the short ends of the reservoir is equipped with one considerably smaller diameter projecting closable spout at which the test device consists of two or more in angle in proportion to each other situated test plates of which at least one in fields are prepared with a culture media and antibiotics of different kind and strength.

### Technical background of the innovation

Analysis of urine is one of the oldest, simplest, and most used laboratory tests in the clinical medicine. The urine should be controlled at each health examination and at each control of a sick patient.

There are a large group of people with no symptoms from the urinary tracts in spite of the fact that they have bacteria in the urine, these patients might also have reduced functions of their kidneys. It is important to make a survey of these patients conditions before a so called Chronically Pyelonephrit arises. Silent urinary tract infections are a major problem of today and it is especially in the non-institutional care that an efficient examination of the urine, by cultivation and resistance tests, are very important.

An ordinary analysis of the urine is always done as a primary check. The analysis comprehends colour and other optical appearance, pH-value, protein, density, sugar, and occurrence of red and white corpuscles. If white corpuscles and/or bacteria are discovered should a urine cultivation be made and it should be combined with a test of bacteria resistance indicating the most convenient antibiotic for treatment.

In the above mentioned technical field exists many different solutions for determination of bacteria-content in urine and many different ways to confidently determine which antibiotic is suitable to ordinate for that specific bacteria.

In a usual procedure the patient hands a urine sample in a disposable glass container or similar. Even if the urine sample have been handed correctly to avoid contamination the sample will always, more or less, be contaminated with other bacteria during transportation to the bacteriological laboratory. The urine is a excellent medium for bacteria growth, which leads to uncertain interpretation of the test results. Contamination can increase the number of bacteria by millions per millilitre of urine in very short time, if the urine is not examined quickly or refrigerated. Without effective refrigeration the maximum time between the urine is handed and when it is taken care of at a laboratory is 1-2 hours, if the reliability of the test-result shall remain undecresed.

With the common routines in the ward of today must the urine sample be send or transported with mail or delivery service to a suitable laboratory where urine samples are kept refrigerated until cultivated and analysed. It normally takes three days before the test result is ready and first after that can suitable treatment be ordinated. This procedure increases the risk for complications for the patient. The bacteria that occurs when cultivated must be resistance tested in order to find out which antibiotic is suitable for treatment. It is important not to take a chance and just choose a antibiotic among all the preparations available in the market. It is therefor important that a resistance test is made as quickly as possible so the most suitable antibiotic can be chosen and determined which strength it should have in order to obtain good results.

Today cultivation at a bacteriological laboratory is made with agar-plates that is moistened with urine and the resistance pattern is determined through a magnifying glass.

The existing but not so commonly used dip-slide method is less expedient in a epidemiological and prognostic point of view.

It is also threw the Swedish patent document SE,C2 503 177 known about a device for resistance testing of micro-organisms in e.g. urine, this device consists of a closable cylindrical reservoir and a test device, which can be put in the reservoir, prepared with a culture media. The test device is situated in the front part of one in the reservoir axially shiftable piston, which makes it watertight against the inner wall of the reservoir, at which one of the short ends of the reservoir is equipped with one with considerably smaller diameter projecting closable spout at which the tests device consists of two or more in angle in proportion to each other situated test plates of which at least one in fields are prepared with a culture media and antibiotics of different kind and strength.

However it has shown that this device can not be used in a sufficient way for correct interpretation of the plates because of mist over the inside of the reservoir. Despite different efforts to remove the mist by e.g. turning the piston have this problem remained and this device have therefore not been able to solve the above mentioned problems with urine testing.

Because of this can the result be misinterpreted and incorrect result is noted.

US-A-4,054,491, discloses a device for testing the presence of microorganisms in a liquid sample, which comprises a syringue-like growth chamber with a sealable spout projecting from one end surface, and a piston having the same outer shape as the inner shape of the growth chamber. The lower end part of the growth chamber (and accordingly the lower part of the piston) can be provided with a conical narrower part that terminates in the spout and which function is to allow a more accurate determination of the volume of sample introduced in the chamber and to form an air cushion over the mixture of sample and culture medium. In this device the sample is introduced together with the culture medium and is not removed from the device during the test measurements.

DE-A- 27 11 201, discloses a device for determining the amount of urea in urine, which comprises a cylindrical holder containing a test strip and a piston for sucking the liquid sample inside the holder. The lower end part of the holder is conical shaped and ends in a spout with the purpose to maintain the test strip into the holder. The wall of the holder is provided at its upper end with an orifice for removing excess of sample which may soak the indicator zone of the test strip, giving rise to false test results.

### Brief account of the innovation aims

The purpose of the existing innovation is to eliminate the above mentioned disadvantages of test devices for urine and to attain a test device that is easy to interpret thanks to the minimised risk for mist over the inside of the reservoir that surrounds the test plates.

One aim for the innovation is to attain a device that indicates if a infection exists and which medication that should be made, everything in one step in a satisfactory way.

Another aim for the innovation is to attain a easily interpreted device.

One aim for the innovation as to attain a test device that completely can be emptied from urine after the plates have been moistened.

One further aim for the innovation is to attain a easily handled and a non-spilling test device.

One further aim for the innovation is to attain a disposable test device with low production costs.

In accordance with the aims of the innovation are the above mentioned problems solved by making the lower end of the mentioned cylindrical reservoir conical with a spout where the conical part passes into the mentioned spout to prevent urine from accumulate at the lower end of the reservoir when the cylindrical reservoir have been emptied from urine after the mentioned plates have been moistened with urine and before the device is placed in a incubator for cultivation.

In accordance with the aims for the innovation is a device obtained that is dependable, labour-saving, low in production costs, and considerably simplifies test-routines.

### Brief description of enclosed drawings

Fig 1 shows the device seen from the side.

Fig 2 shows that part of the device which shows the test-plates and the lower part of the reservoir, seen enlarged from the side.

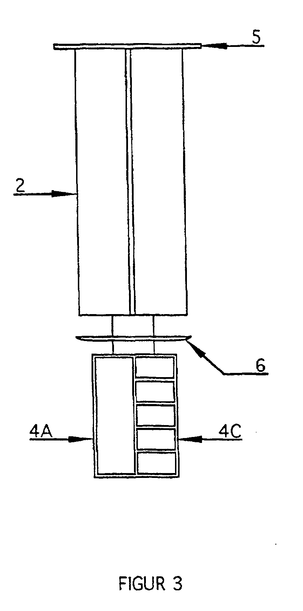

Fig 3 shows the piston with test-plates, seen from the side.

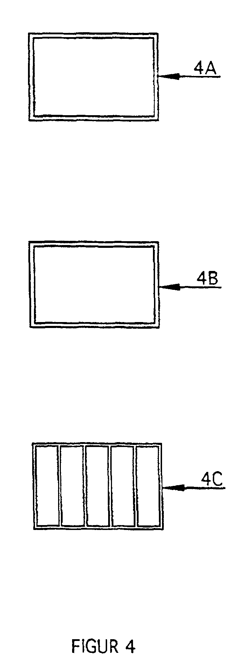

Fig 4 shows the test-plates with culture media for e.g. antibiotics of different types and strength.

### Detailed description of a preferred design of the innovation

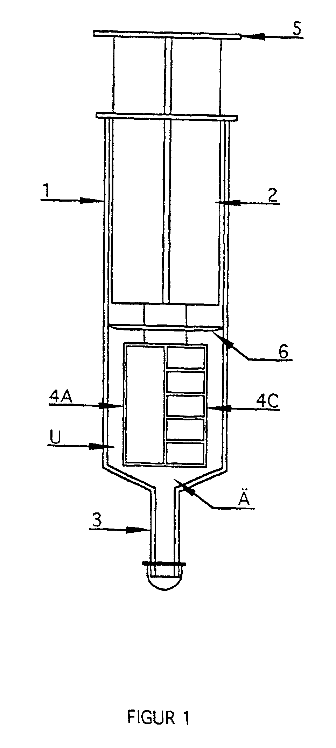

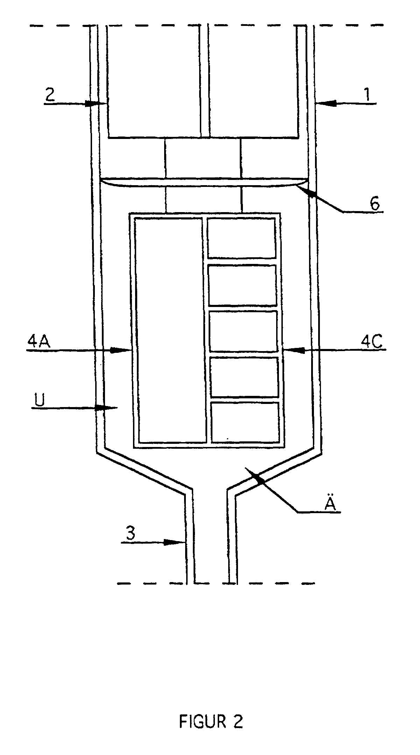

Figures 1 and 2 shows the innovation device, that looks like a syringe, including a piston 2 with one in one end arranged plate for handling and placement in a incubator 5, where the piston 2 is put inside in a partly or fully transparent cylindrical reservoir I which is open in one end and in the other end equipped with a closable spout 3.
At the inner end, when inserted, is the piston 2 equipped with a number of test-plates 4a, 4b and 4c some coated solely with culture media and some with both culture media and antibiotics. On the piston 2 is furthermore a radially disc-shaped slightly deformable sealing 6 placed between the test-plates and the outer part of the piston. The sealing 6 diameter is slightly larger then the inner diameter of the cylindrical reservoir. When the piston is inserted in the cylindrical reservoir will the sealing 6 slightly be deformed which makes it water-tight against the inner walls of the reservoir between the lower chamber of the cylindrical reservoir with its test-plates and the upper open chamber of the cylindrical reservoir.

The lower end Ä of the cylindrical reservoir 1 is conical shaped with the closable spout 3 placed around the top of the cone to prevent accumulation of urine there when the cylindrical reservoir I is emptied from urine after the test-plates 4a, 4b, and 4c have been moistened with urine.

The conical end has an angle between 3 and 30°. The spout 3 diameter is approximately one ninth of the inner diameter of the cylindrical reservoir 1 in order to minimise the surface on which urine can accumulate after the cylindrical reservoir has been emptied from urine and in that way minimise the risk of mist on that part of the cylindrical reservoir that surrounds the test-plates 4a, 4b, and 4c.

At the upper outer part of the piston is a plate for handling and placement 7 arranged on which the device stands during incubation.

The innovation is delivered to users coated from the manufacturing site which means that the test-plates have been equipped with culture media respective culture media and antibiotics in layers of about 2,3 mm in thickness after which the piston 2 with the test-plates is inserted into the cylindrical reservoir 1 and the sealing 6 makes it tight against the inner walls of the reservoir 1 after which the device is sterilised and packaged.

A relatively thick layer of agar is preferred when this is a perishable and stays fresh for a longer time if the layer is about 2,3 mm in thickness and in that way does not dry out so fast.

The plates can be equipped with a check pattern to make it easier for the agar to get a hold on the plates.

When the device according to the innovation is to be used is the package opened and the sealing over the spout 3 is removed and the spout 3 is dipped into a container filled with liquid e.g. urine that should be tested. The piston 2 is pulled a short way inside the reservoir 1, in direction out of the reservoir 1, until sufficient volume of urine have been sucked into the reservoir 1 after which the device is easily shaken in order to moisten the test-plates 4a, 4b, and 4c with the urine, after which the piston 2 is fully pushed into the reservoir 1 at which all urine spurts out through the spout 3 after which the spout 3 is closed. The sealing 6 makes it water-tight against the inner wall of the cylindrical reservoir 1 and surrounds the test-plates 4a, 4b, and 4c in a space U between this sealing 6 and the closable spout 3.

It is important that no surplus of urine is left in this space U, when this can cause mist on the inner walls of the cylindrical reservoir 1 and make it impossible to interpret the results.

The device is now ready for placement in a warm atmosphere e.g. in a incubator, preferably in 37° C, for cultivation. The device is placed with the spout upwards standing on the plate for handling and placement 5.

After a certain time, preferably after 4-8 hours, are growth of bacteriacultures interpreted at the same time as it one the plates with antibiotics directly, without microscope or magnifying glass, can be seen on which plates bacteria has not succeeded to grow and from that decide which medication and/or which medication strength that is suitable in this specific case.

With the innovation can e.g. a private practitioner without full laboratory equipment easily establish and treat different kind of urinary tract infections.

## Claims

1. A device for resistance tests of e.g. in urine occuring micro-organisms especially bacteria which consists of a closable cylindrical reservoir (1) and one in that insertable piston-shaped part (2) coated with culture media, at which the coated part (2) is arranged in the front part of the in the reservoir axially shiftable piston-shaped part which makes it water-tight against the inner walls of the reservoir and at which one of the short ends of the reservoir is equipped with one with considerably smaller diameter projecting closable spout (3) and the other end is closable with a in a radially direction stretchable disc-shaped sealing (6) at which the coated part (2) consists of three or more in angle in proportion to each other situated test plates (4a, 4b, 4c) of which at least one in fields are coated with culture media and antibiotics of different type and strenght, **charac terised by** that the lower end part (Ä) of the cylindrical reservoir (1) is conical shaped with a tip where the conical part passes into the spout (3) to prevent accumulation of urine at this end part (Ä) when the cylindrical reservoir (1) is emptied from urine after the test plates (4a, 4b, 4c) have been moistened with urine and before the device is placed in a incubator for cultivation.

2. A device according to the claim 1, is **characterised by** that the angle of the conical end part (Ä) is between 3 and 30°.

3. A device according to the claim 1, is **characterised by** that the diameter of the spout (3) is approximately one ninth of the inner diameter of the cylindrical reservoir (1).

## Patentansprüche

1. Anordnung für Resistenztests von bspw. im Urin vorkommenden Mikroorganismen, speziell Bakterien, welche Anordnung aus einem verschließbaren zylindrischen Behälter (1) und einem in diesen einsetzbaren kolbenförmigen Teil (2) besteht, der mit einem Kulturmedium beschichtet ist, wobei der beschichtete Teil (2) im vorderen Bereich des im Behälter axial verschiebbaren kolbenförmigen Teils angeordnet ist, der ihn wasserdicht gegen die Innenwandflächen des Vorratsbehälters abschließt, wobei eines der kurzen Enden des Behälters mit einem mit erheblich kleineren Durchmesser vorstehenden verschließbaren Ausfluss (3) versehen und das andere Ende mit einer in einer radialen Richtung streckbaren scheibenförmigen Dichtung (6) verschließbar ist, wobei der beschichtete Teil (2) aus drei oder mehr im Winkel proportional zueinander angeordneten Testplättchen (4a, 4b, 4c) besteht, von denen mindestens eines in Feldern mit Kulturmedien und Antibiotika unterschiedlicher Art und Stärke beschichtet ist, **dadurch gekennzeichnet, dass** der untere Endteil (Ä) des zylindrischen Behälters (1) konisch geformt ist mit einer Spitze, wo der konische Teil in den Ausfluss (3) übergeht, um Urinansammlungen an diesem Endteil (Ä) zu verhindern, wenn der zylindrische Behälter (1) von Urin entleert wird, nachdem die Testplättchen (4a, 4b, 4c) mit Urin befeuchtet wurden und bevor die Anordnung zum Kultivieren in einen Inkubator eingebracht wird.

2. Anordnung nach Anspruch 1, **dadurch gekennzeichnet, dass** der Winkel des konischen Endbereichs (Ä) zwischen 3° und 30° liegt.

3. Anordnung nach Anspruch 1, **dadurch gekennzeichnet, dass** der Durchmesser des Ausflusses (3) etwa ein Neuntel des Innendurchmessers des zylindrischen Behälters (1) beträgt.

## Revendications

1. Dispositif pour effectuer des tests de résistance de par exemple des micro-organismes apparaissant dans l'urine, en particulier des bactéries, ce dispositif consistant en un réservoir cylindrique pouvant être fermé (1) et une partie en forme de piston (2) pouvant être introduite dans le réservoir (1), cette partie (2) étant revêtue d'un milieu de culture, la partie revêtue (2) étant disposée dans la partie avant du réservoir de façon que la partie en forme de piston déplaçable axialement assure l'étanchéité à l'eau contre les parois intérieures du réservoir, tandis que l'une des extrémités courtes du réservoir est équipée d'un goulot en saillie (3) pouvant être fermé et présentant un diamètre beaucoup plus petit, l'autre extrémité pouvant être fermée par un joint d'étanchéité en forme de disque (6) étirable radialement, la partie revêtue (2) étant constituée de trois plaques de test (4a, 4b, 4c) ou plus formant des angles les unes avec les autres, l'un au moins des domaines de ces plaques étant revêtu d'un milieu de culture et d'antibiotiques de différents types et de différentes forces,
**caractérisé en ce que**
la partie d'extrémité inférieure (Ä) du réservoir cylindrique (1) est de forme conique avec un bout par lequel la partie conique passe dans le goulot (3) pour éviter l'accumulation d'urine à l'endroit de cette partie d'extrémité (Ä) lorsque le réservoir cylindrique (1) est vidé d'urine après que les plaques de test (4a, 4b, 4c) aient été humidifiées d'urine, et avant que le dispositif soit placé dans un incubateur pour effectuer la culture.

2. Dispositif selon la revendication 1,
**caractérisé en ce que**
l'angle de la partie d'extrémité conique (Ä) est compris entre 3° et 30°.

3. Dispositif selon la revendication 1,
**caractérisé en ce que**
le diamètre du goulot (3) est d'approximativement le neuvième du diamètre intérieur du réservoir cylindrique (1).
